# EUROPEAN PATENT APPLICATION

(11) **EP 1 212 982 A2**
(43) Date of publication of application: **12.06.2002**
(21) Application number: 02001557.4
(22) Date of filing: 21.03.1998
(51) Int. Cl.: A61B 10/00

(54) **Biopsy surgical appliance**

(30) Priority: 03.04.1997 IT BO970195
(62) Divisional of application: 98105158.4
(71) Applicant: Allegiance Healthcare Corporation, McGaw Park, IL 60085 (US)
(72) Inventor: Bauer, Alberto, Santo Domingo (DO)
(74) Representative: Gustorf, Gerhard, Dipl.-Ing.

(57) **Abstract**

A biopsy surgical appliance comprises a needle for biopsy consisting of two components called stylet (21) and cannula (31), which are disposed coaxially along an axis (Y) and are activated by a mechanical device with two slides (20, 30) which can slide longitudinally inside a shell (10, 50). The slide (30) which supports the rear portion of the cannula (31) comprises an extension (35) which projects outside the shell (50) and slides longitudinally along a through-slot (71) provided in one wall of said shell (50), in order to be able to withdraw said second slide (30) from the exterior, by acting on a pawl (80) which is disposed at the free end of said extension (35) in order to uncover a recess (29) of the stylet (21) in which the sample of tissue collected is accommodated.

## Description

### BACKGROUND OF THE INVENTION

### Field of the Invention

The present invention relates to a biopsy surgical appliance.

Biopsy surgical appliances are commonly used to remove a sample of tissue from inside a living being.

At present, various biopsy surgical appliances are known, which substantially comprise a needle for biopsy, consisting of a stylet and a cannula which extend coaxially, the rear portions of which are attached to a mechanical device which is disposed inside a shell, such that they can be activated automatically or semi-automatically.

In appliances of this type, the stylet consists of a thin probe, the front end of which is pointed and is preceded by a recess for accommodation of the sample to be collected. The cannula consists of a tube which is fitted coaxially onto the said stylet, slides axially relative to the stylet, and has a cutting front end which is designed to close the recess provided in the front portion of the stylet.

The mechanical device substantially comprises two slides which can slide longitudinally inside the said shell, resilient means designed to load the said two slides resiliently and longitudinally, coupling/release means which are designed to retain the said two slides in the armed position and to release them, loading means which are designed to bring the said two slides into the armed position, and control means to release the said two slides from their armed position: in order to be able to set this appliance in the armed position, insert the front end of the needle for biopsy in the vicinity of the tissue to be collected, and to carry out the collection by advancing in respective automatic or semi-automatic succession first the stylet and then the cannula.

By way of example see patents US-4.958.625, EP-0.536.888, WO-91/01112 and US-4.924.787.

The known biopsy surgical appliances have some disadvantages.

A first disadvantage consists of the fact that they are very heavy, such that during use, the operator, must support the appliance continuously in order to eliminate undesirable advance movements and/or displacements caused by the action of the force of gravity.

A second disadvantage is caused by the fact that the said appliances are complex to manoeuvre, and for example do not allow the operator to control and/or analyse and/or remove the sample easily after the needle has been extracted from the body of the living being.

A third disadvantage consists of the fact that the said appliances are very costly, owing to a large number of components, and because of the complex structuring of the mechanical device which requires considerable assembly times.

### SUMMARY OF THE INVENTION

The aim of the present invention is to eliminate the above-described disadvantages. The invention, which is characterised by the claims, solves the problem of creating a biopsy surgical appliance.

The object of the present invention is thus a biopsy surgical appliance, comprising a needle for biopsy consisting of two components called stylet and cannula which are disposed coaxially and extend axially along an axis, and of a shell to be grasped, in which the said needle for biopsy has a free front portion which is designed to be inserted in the collection area, and another, rear portion which is associated with a mechanical device for automation of the collection operations and is disposed inside the said shell, in which the said mechanical device has two slides which can slide longitudinally inside the said shell, and are designed to support the rear portions of the said stylet and of the said cannula, in which there are provided resilient means which are designed to load resiliently and longitudinally the said two slides, in which there are provided coupling/release means designed to retain in the armed position and release the said two slides, in which there are provided loading means designed to bring the said two slides into the armed position, and in which there are provided control means to release the said two slides from their armed position, in which the said biopsy surgical appliance is characterised in that the first slide of the said two slides has a "U" shape with tines which are oriented longitudinally and is moved longitudinally in a first operative environment; in that the central section of the said "U" shape supports the rear portion of one component of the said two components which constitute the needle for biopsy; in that the second slide of the said two slides has a head which extends towards and beyond the said axis without interfering with the said first operative environment of the first slide and is inserted freely between the tines of the said first slide; and in that the said head supports the rear portion of the other component of the said two components which constitute the needle for biopsy.

The following results are obtained by means of the biopsy surgical appliance which is the subject of the present invention: an improvement in use, since it is lighter; an improvement in the post-collection operations, the purpose of which is to check and/or analyse and/or remove the sample; and a reduction in the manufacturing costs.

Further features and advantages of the present invention will become more apparent from the following detailed description of a preferred practical embodiment, provided here purely by way of non-limiting example, with reference to the figures of the attached drawings in which:

### BRIEF DESCRIPTION OF THE DRAWINGS

Figure 1 is an exploded view of the components of the biopsy surgical appliance which is the subject of the present invention;
Figure 1A is a view from below of the upper half-shell of the said appliance;
Figure 1 B is a plan view of the lower half-shell of the said appliance;
Figure 1C is a cross-sectional view along the vertical median longitudinal plane, showing the two half-shells in figures 1A and 1B joined;
Figure 1D is a view in cross-section along line D-D in figure 1C, showing the two half-shells in figures 1A and 1B joined;
Figure 2 is a perspective view showing the said appliance in a position of rest, with the upper half-shell in figure 1A partially removed;
Figure 2A is a cross-sectional view along the vertical median longitudinal plane, of the appliance illustrated in figure 2 with the upper half-shell;
Figure 2B is a schematic plan view of the appliance illustrated in figure 2;
Figure 3 is a perspective view illustrating the said appliance in an armed position, with the upper half-shell in figure 1A partially removed;
Figure 3A is a cross-sectional view along the vertical median longitudinal plane, of the appliance illustrated in figure 3, with the upper half-shell;
Figure 4 is a perspective view illustrating the said appliance in a position with the stylet advanced, with the upper half-shell in figure 1A partially removed;
Figure 4A is a cross-sectional view along the vertical medial longitudinal plane of the appliance illustrated in figure 4, with the upper half-shell;
Figure 5 is a perspective view illustrating the said appliance in a specific cross-sectional position, with the upper half-shell in figure 1A partially removed;
Figure 5A is a cross-sectional view along the vertical medial longitudinal plane of the appliance illustrated in figure 5, with the upper half-shell;
Figure 6 is a perspective view illustrating the said appliance in a specific position which is designed to show the sample taken, with the upper half-shell in figure 1A partially removed: and
Figure 7 illustrates schematically a variant embodiment of the present invention.

### DESCRIPTION OF THE PREFERRED EMBODIMENT

With reference to 1, 1A, 1B, 1C, 1D, 2, 2A and 2B, the biopsy surgical appliance is symmetrical relative to the vertical longitudinal median plane which is disposed along the longitudinal axis Y, and consists of various components.

A first, upper half-shell 10, with lateral retention walls 11a, 11b, a front wall 11f and a rear wall 11p, in which the rear wall 11p has two pins 12a and 12b designed to bring the rear end of two respective springs 13a and 13b; and the front wall 11f has at the front a pair of stop shoulders 14a and 14b with stop surfaces 15a, 15b, and a tube means 16 which allows the needle for biopsy to emerge. -In addition, longitudinally and centrally, there is provided a guide channel 17 which is open at the rear, for the reasons described hereinafter.

A first slide 20, designed to bring integrally a stylet 21 which is provided with a known recess 29 for accommodation of the sample collected, in which the said first slide 20 has a horizontal "U" shape, the tines 22a, 22b of which are oriented longitudinally, and project towards the front of the appliance, and the central section 22c of which brings integrally in its centre the stylet 21.

In addition, for the reasons which will become apparent hereinafter, the said first slide 20 has: two upper teeth 23a, 23b; two front stop surfaces 24a and 24b; two front slider feet 25a and 25b which are disposed at the respective transverse ends, and have a calibrated transverse amplitude; two rear slider feet 27a and 27b (only the 27a can be seen); and a connection surface 26 which is disposed between the said tines 22a and 22b.

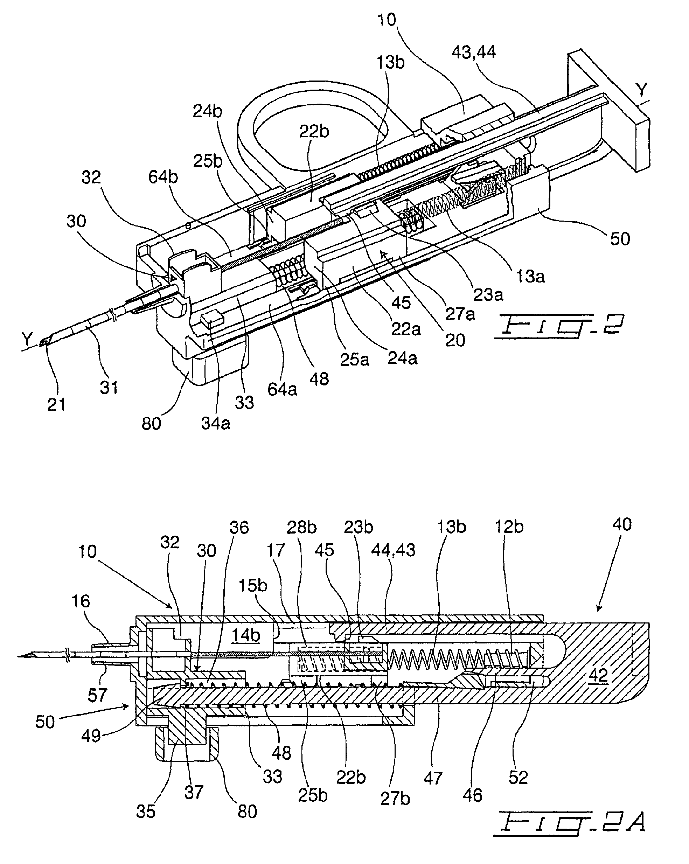

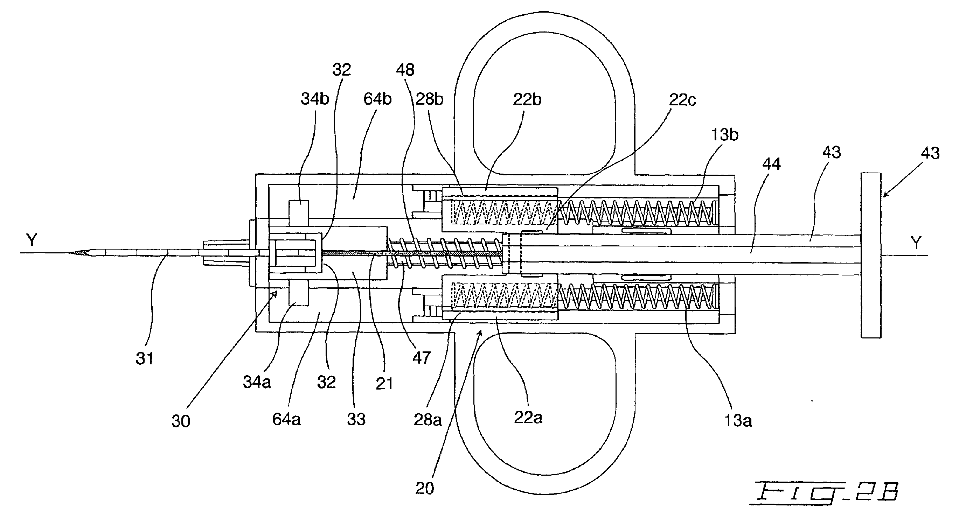

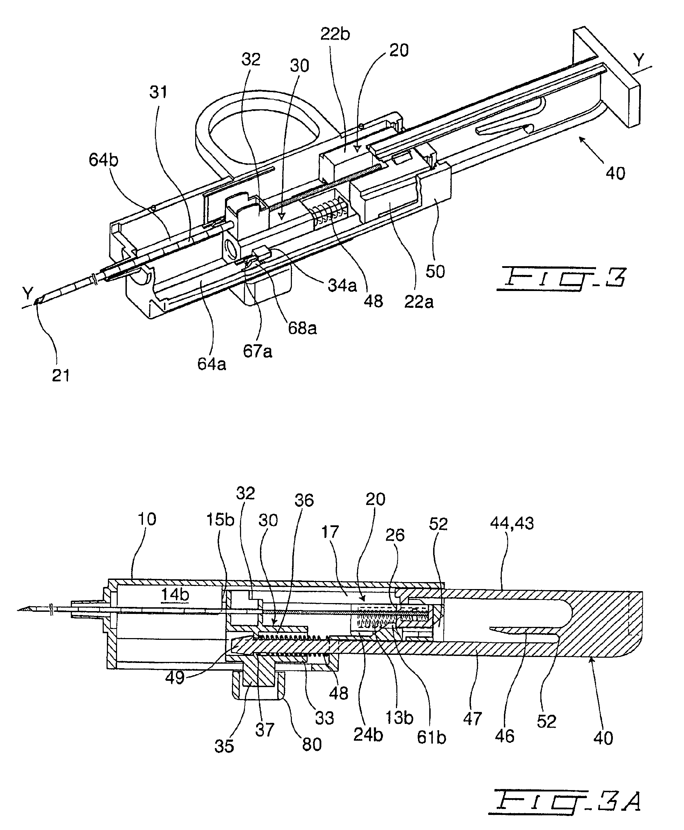

A second slide 30, which, for the reasons which will become apparent hereinafter has: a head 32 which is designed to bring integrally a cannula 31, and has a shape such that it can be inserted in a sliding manner between the tines 22a and 22b of the said first slide 20 (see also figure 4); a central body 33 which has fins 34a, 34b which project transversely; an extension 35 which projects from the exterior of the shell in order to be connected to a pawl 80, as described in greater detail hereinafter; and a longitudinal central through-hole 36 which has a choke 37.

A loader and activator component 40, which is designed to set the two slides 20 and 30 in the armed position, and to activate them for the collection, which component is three-pronged, and has: a grasping head 41 with grasping wings 41a and 41b; a central part 42; a first, upper branch 43 which has an upper longitudinal profile 44 which can slide inside the channel 17 of the first half-shell 10 and a drawing tooth 45 which can engage the teeth 23a, 23b of the first slide 20; a second, short intermediate branch 46 which has a wedge-shaped tapered end; and a third branch 47, which is designed to bring a spring 48 which is fitted onto the latter, and has an engagement head 49 at its free end.

A second, lower half-shell 50, with lateral retention walls 51a, 51b, a front wall 51f and a rear wall 51p, in which the rear wall 51p has a square through-hole 52; the two lateral walls 51a, 51b have two outer rings 55a, 55b which are designed to act as grasping components for the operator's fingers; and the front wall 51f has a tube means 57 which is designed to permit sliding of the needle for biopsy.

In its rear part, the base of the said second half-shell 50 has a first automatic coupling 58, which consists of a flexible tab 59 which has two lateral shoulders 60a, 60b, which form two retention teeth 61a, 61b and two support projections 73a, 73b.

Below the said flexible tab 59, towards the front of the appliance, there is a longitudinal channel 62 which is open at the top, the rear wall 53 of which has a through hole 63.

By means of this structuring, the said second half-shell 50 forms two lower sliding planes, i.e. the first 64a, 64b, which is disposed at the side and at the level of the top of the channel 62; and the second 65a, 65b, on the base of the said central channel 62, in which this latter plane 65a, 65b has a longitudinal through-slot 71, for the reasons which will be described hereinafter.

The two sliding planes 64a, 64b have respective second coupling/release means 74a, 74b, each of which consists of a flexible tab 66a, 66b which has two projections 67a, 68a, 67b, 68b, in which the projections 67a, 67b have coupling teeth 70a, 70b, and in which the projections 68a, 68b have inclined planes 69a, 69b which are oriented from the exterior towards the interior, and from the rear towards the front of the shell 50, for the reasons which will become apparent hereinafter. In addition, respective intermediate stop teeth 72a, 72b are provided along the said sliding planes 64a, 64b.

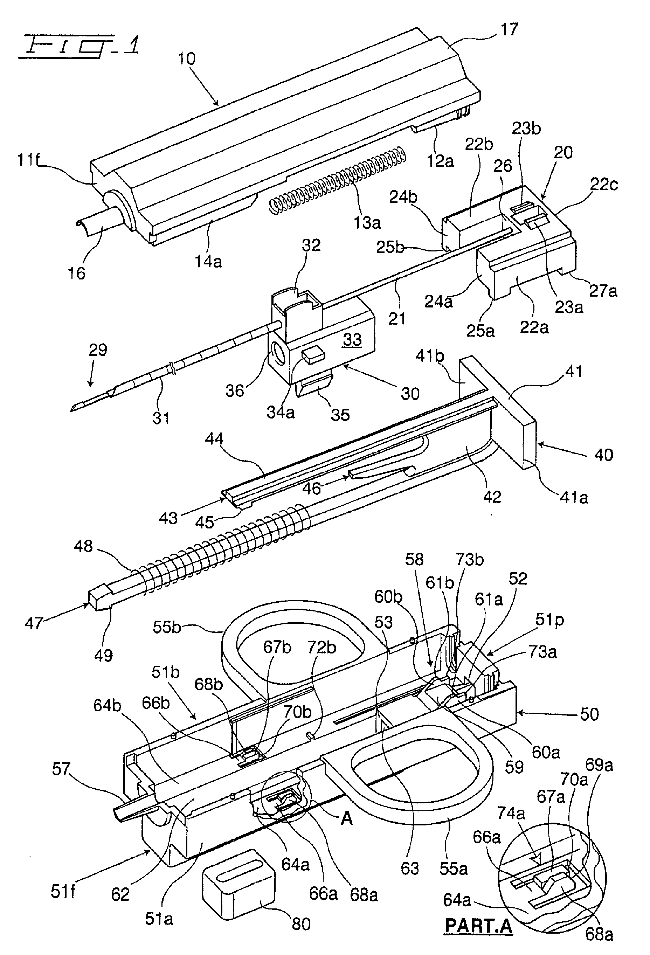

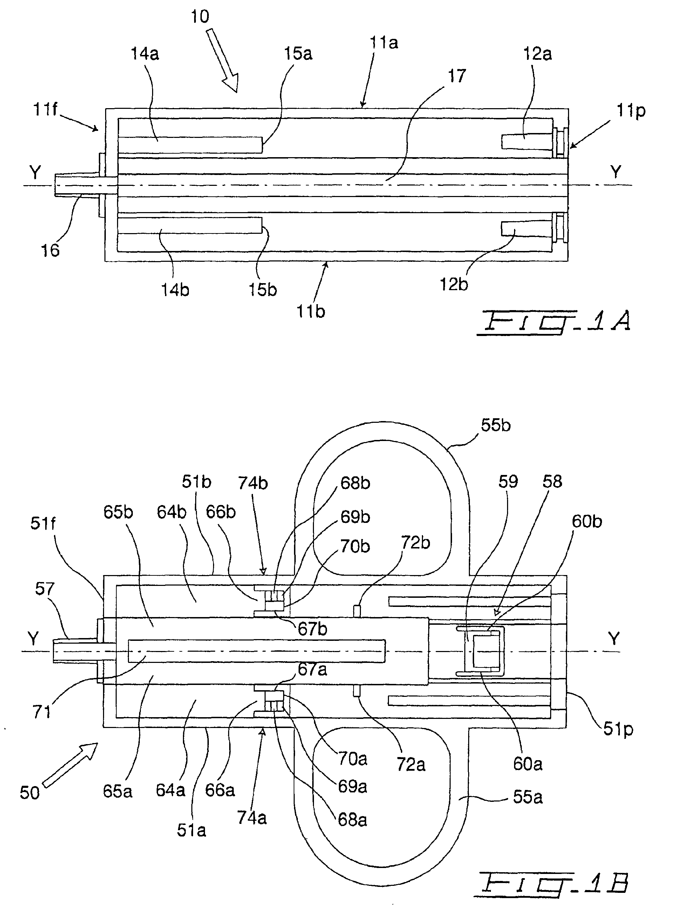

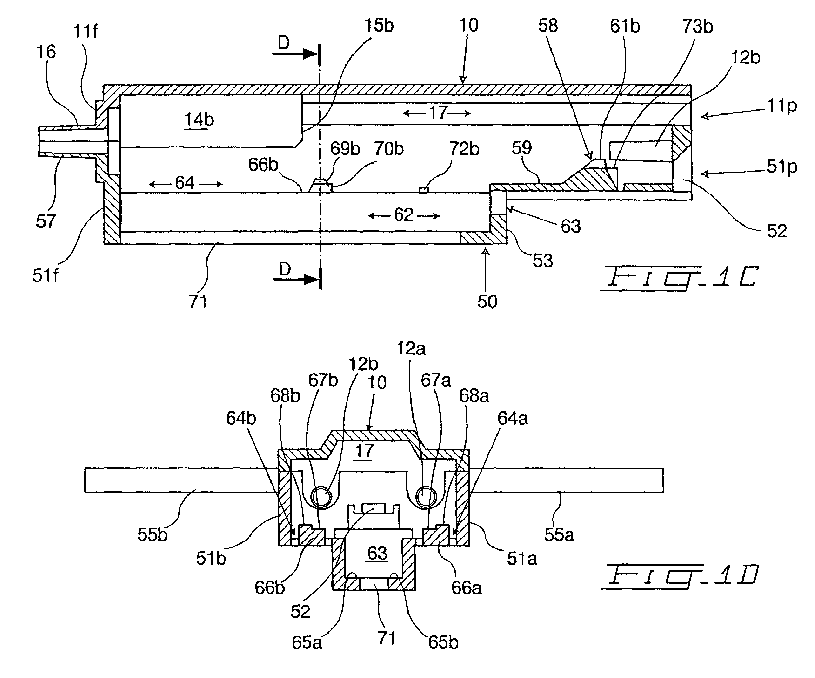

With reference to figures 2 and 2A, when the various components are assembled and are disposed inside the upper half-shell 10 and the lower half-shell 50, the first slide 20 has its own feet 25a, 25b, 27a, 27b supported in a sliding manner on the sliding planes 64a, 64b, and the said first slide 20 is also thrust forwards by the springs 13a, 13b which have one end fitted onto the pins 12a, 12b of the half-shell 10, and the other end accommodated in blind holes 28a, 28b which are oriented axially, and are provided in the rear part of the said first slide: see also figure 2B.

The second slide 30 is disposed with its lower part 33 partially inside the sliding channel 62, and has its fins 34a, 34b supported in a sliding manner on the sliding planes 64a, 64b.

The loader/activator component 40 is disposed at the rear and has: the first branch 43, with the corresponding profile 44, inserted longitudinally in a guided sliding manner along the channel 17 of the first half-shell 10, with the entrainment tooth 45 in the vicinity of the teeth 23a 23b of the first slide 20; the second branch 46, which is inserted longitudinally in a sliding manner through the aperture 52, with its wedge-shaped free end inserted between the shoulders 60a 60b of the first flexible coupling 58; and the third branch 47 inserted longitudinally in a sliding manner through the aperture 63, with its head 49 disposed beyond the choke 37 provided in the hole 36.

The spring 48 is fitted onto the said branch 47, has its front part partially disposed inside the hole 36, and its opposite ends disposed against the rear surface of the choke 37 and against the front surface of the rear wall of the channel 62, in order to thrust the said second slide 30 forwards.

In order to set the said appliance in the armed position, the loader/activator component 40 is drawn rearwards, which action draws the first slide 20 rearwards by the interception of the tooth 45 against the teeth 23a 23b, and draws the second slide 30 rearwards by interception of the head 49 against the front wall of the choke 37.

Figures 3 and 3A illustrate the configuration of the appliance after the action of loading or arming: in which the slide 20 is coupled in the armed position, with the springs 13a, 13b which are compressed, since the coupling teeth 61a, 61b are coupling the front wall 26 of the said first slide 20; and in which the slide 30 is coupled in the armed position with the spring 48 compressed, since the coupling teeth 70a, 70b are coupling the front surface of the fins 34a, 34b.

In this configuration, the needle for biopsy is inserted in the patient in order to position it in the vicinity of the area where the collection is to be carried out.

When the needle is in the correct position (see also figures 4 and 4A), the operator advances the loader/activator component 40 and the branch 46 enters inside the shell via the aperture 52.

Further advance of the loader/activator component 40 gives rise to infra-positioning of the wedge-shaped end of the branch 46 between the flexible tab 59 and the central section 22c of the first "U"-shaped slide 20, with consequent displacement towards the exterior of the free end of the said tab 59 and of the corresponding coupling teeth 61 a, 61 b, until release or uncoupling takes place of the said first slide 20, which, thrust by the springs 13a, 13b and sliding with its own feet 25a, 27a, and 25b, 27b on the respective sliding planes 64a, 64b, moves forwards, giving rise to advance of the tip of the stylet 21 which perforates the tissue to be collected.

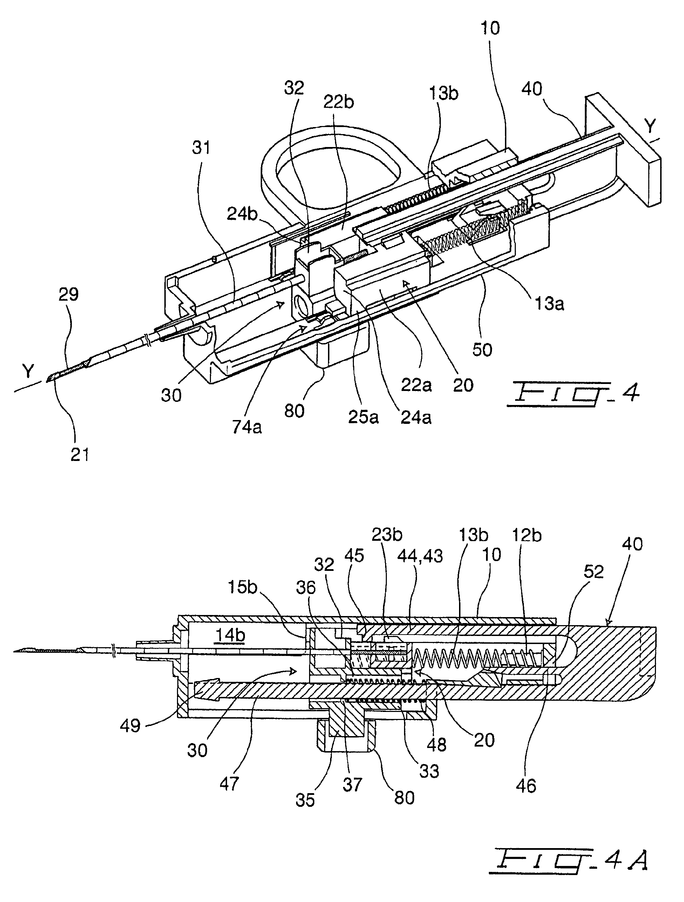

Owing to their transverse arrangement and transverse amplitude, when they move forwards, the front feet 25a, 25b of the first slide 20 pass to the side of the fins 34a 34b and to the said of the coupling teeth 70a 70b, and shortly before the first slide 20 ends its forward path, which terminates when the front surfaces 24a, 24b abut the rear surfaces 15a, 15b of the shoulders 14a, 14b, and the front surface of the rear feet 27a, 27b abuts the rear surfaces of the intermediate stop teeth 72a, 72b, the said front feet 25a, 25b engage with the respective inclined planes 69a, 69b of the projections 68a, 68b, giving rise to flexure towards the exterior of the free ends of the flexible tabs 66a, 66b, with consequent displacement towards the exterior of the coupling teeth 70a, 70b, and corresponding release or uncoupling of the slide 30, which, thrust by the spring 48, begins its path of advance towards the front, advancing the cutting end of the cannula 31, which covers/closes the recess 29, separating/isolating the piece of tissue which is accommodated in the said recess 29, from the remainder of the surrounding tissue: see also figures 5 and 5A.

With reference to the end of advance travel of the first slide 20, caused by the rear feet 27a, 27b meeting the intermediate stop teeth 72a, 72b, it must be pointed out that the said rear feet 27a, 27b have a greater transverse amplitude than the front feet 25a, 25b, and whilst the said first slide 20 is being advanced by the springs 13a, 13b, owing to their smaller transverse amplitude the front feet 25a, 25b move to the side of the said intermediate stop teeth 72a, 72b, whereas owing to their greater transverse amplitude the rear feet 27a, 27b interfere with the same intermediate stop teeth 72a, 72b, thus activating the said end of advance travel function.

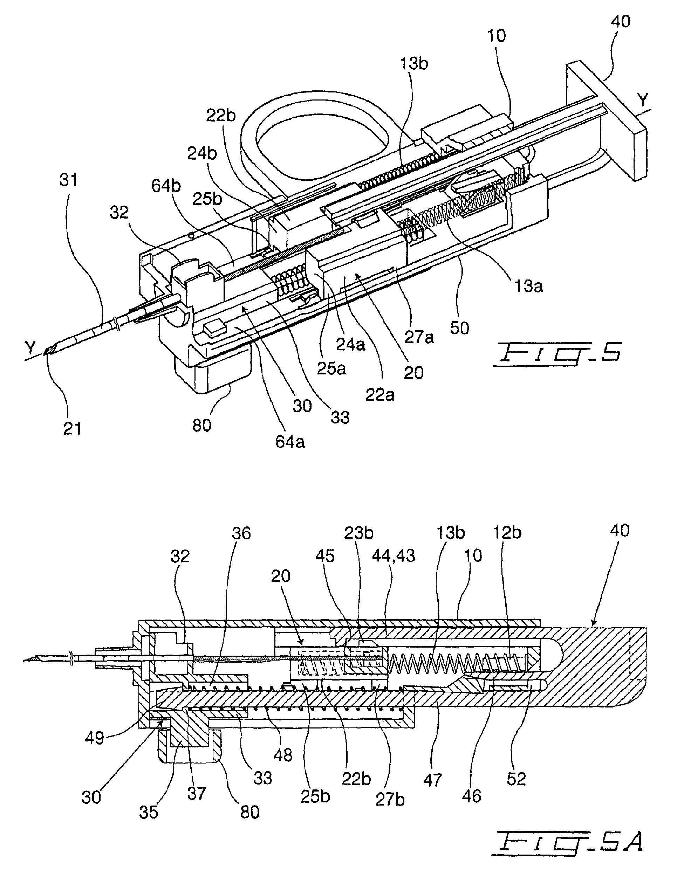

When the sample has been isolated, the needle can be extracted from the body of the living being, and if the operator wishes to inspect immediately the sample collected, if the operator presses the pawl 80 as illustrated in figure 6, when the movement rearwards has been completed, he can easily and immediately uncover/bare the recess 29 of the stylet 21, and expose the corresponding sample accommodated there, since rearward movement of the said pawl 80, which is associated with the second slide 30 by means of the projection 35, gives rise to rearward movement of the tube 31 which is attached to the said second slide 30.

More specifically, uncovering/baring the recess 29, with consequent exposure of the sample, can be of two types, i.e. temporary, if the rearward displacement of the pawl 80 has a path such that it does not take the fins 34a, 34b of the second slide 30 beyond the coupling teeth 70a, 70b; or permanent, if the rearward displacement of the pawl 80 has a path such that it takes the fins 34a, 34b of the second slide 30 beyond the rear end of the coupling teeth 70a, 70b, with consequent coupling.

The said first, temporary configuration can be used for example in order to examine whether the collection has taken place satisfactorily. The said second, permanent configuration can be used for example in order to remove the sample obtained, easily from the recess 29.

With reference to the specific configuration of the two slides 20 and 30, in which the head 32 of the first slide 30 is designed to be inserted between the tines 22a, 22b of the other slide 20, it is apparent that this technical/functional arrangement permits reduction of the length of the shell, compared with the previous embodiments in which the slides were disposed one behind the other, which arrangement provides a consequent reduction of the weight of the said shell.

When the needle has been extracted from the patient, with particular reference to the slide 30 of the cannula 31, to the projection 35 which emerges from the shell 50, and to the pawl 80, it is apparent that an operator can quickly and easily expose, and thus check the sample obtained, by means of simple and natural displacement rearwards of the said pawl 80.

In addition, the operator can choose whether to uncover/bare the collection aperture 29 in order to examine the sample, and then re-close it /cover it, or to uncover/bare the said aperture 29 permanently.

Finally, with particular reference to the structure of the activating device, the components of the latter are decidedly smaller and lighter than the previous embodiments, with consequent reduction of the costs of production and weight of the biopsy surgical appliance.

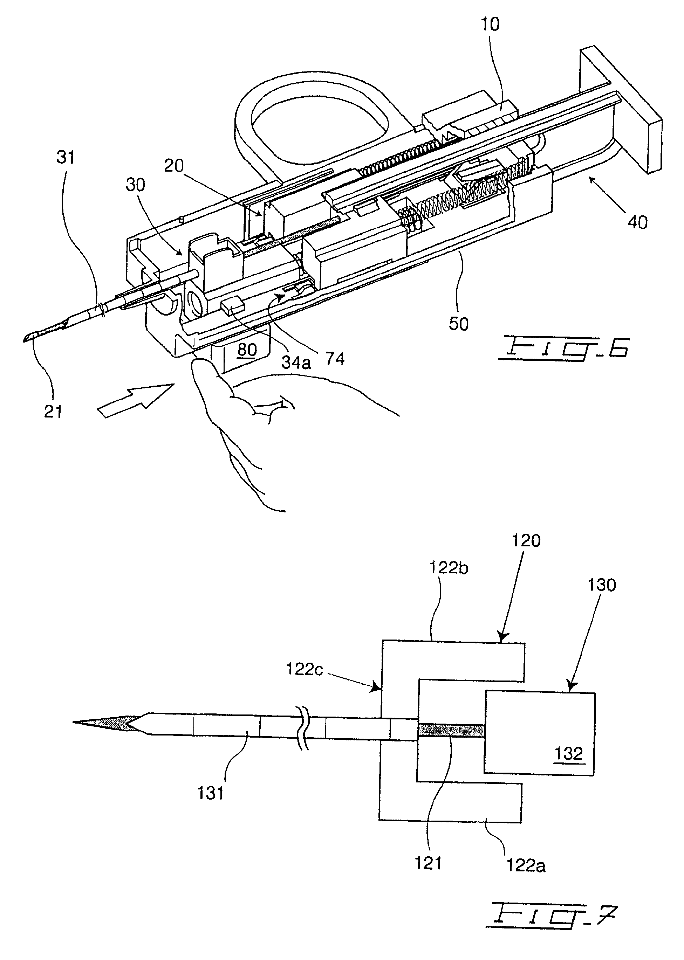

Figure 7 illustrates a variant embodiment of the present invention, which is equivalent to that described above.

In this embodiment, see figure 7, a first slide in the shape of a "U", with tines 122a and 122b, and a central section 122c, supports the rear portion of a cannula 131, and a slide with a head 132, in which the latter has a shape such that it can be inserted between the tines 122a, 122b of the said "U", and supports a stylet 121.

The predetermined objectives are also obtained by means of this equivalent embodiment.

The description of the biopsy surgical appliance which is the subject of the present invention, is provided purely by way of non-limiting example, and it is thus apparent that there can be made to it all modifications or variants which are suggested by practice or by its utilisation or use, provided that these are within the context of the following claims.

## Claims

1. Biopsy surgical appliance comprising a needle for biopsy consisting of two components called stylet (21) and cannula (31) which are disposed coaxially and extend axially along an axis (Y), and of a shell (10, 50) to be grasped, in which said needle for biopsy has a free front portion which is designed to be inserted into the collection area, and another, rear portion which is associated with a mechanical device for automation of the collection operations and is disposed inside said shell (10, 50), in which said mechanical device has two slides (20, 30) which can slide longitudinally inside the shell (10, 50) and are designed to support the rear portions of said stylet (21) and of said cannula (31), in which there are provided resilient means which are designed to load resiliently and longitudinally said two slides (20, 30), in which there are provided coupling/release means designed to retain in the armed position and release said two slides (20, 30), in which there are provided loading means designed to bring said two slides (20, 30) into the armed position, and in which there are provided control means to release said two slides (20, 30) from their armed position, **characterised in that** the slide (30) which supports the rear portion of the cannula (31) comprises an extension (35) which projects outside the shell (50) and slides longitudinally along a through-slot (71) provided in one wall of said shell (50), in order to be able to withdraw said second slide (30) from the exterior, by acting on a pawl (80) which is disposed at the free end of said extension (35), in order to uncover a recess (29) of the stylet (21) in which the sample of tissue collected is accommodated.

2. Biopsy surgical appliance according to claim 1, **characterised in that** the path of withdrawal of said slide (30) of the cannula (31) has an amplitude such that said slide (30) can optionally be disposed in the armed position by being coupled to the respective coupling means (68a, 68b).
